# EUROPEAN PATENT APPLICATION

(11) **EP 1 502 910 A1**
(43) Date of publication of application: **02.02.2005**
(21) Application number: 04254278.7
(22) Date of filing: 16.07.2004
(51) Int. Cl.: C07C 67/08, C07C 69/26, C07C 69/14, C07C 69/24, C07C 69/80

(54) **Methods for esterification in the presence of a sulfonated, halogenated and base treated styrene DVB copolymer catalyst.**

(30) Priority: 29.07.2003 US 490882
(71) Applicant: Rohm and Haas Company, Philadelphia, Pennsylvania 19106-2399 (US)
(72) Inventor: Ramprasad, Dorai, Allentown, Pennsylvania 18104 (US); Collin, Jennifer Reichl, Devon, Pennsylvania 19333 (US)
(74) Representative: Kent, Venetia Katherine

(57) **Abstract**

The present invention provides a system, method and catalyst for esterification. The method of esterification includes adding to reactants of a reaction a base treated, sulfonated, halogenated thermally stable catalyst. The catalyst provides high selectivity and does not readily degrade at high temperatures. The esterification reaction are used to prepare an acetate ester, an acrylic ester, a natural fatty acid ester, a sorbitol derived ester, a phthalate, and/or a benzoate and ester derived from maleic anhydride. A system for esterification, and ester utilizing the method are also provided. Various downstream products are made from the esters created using the method described herein are made including a solvent for a surface coating, an ink, a textile, an adhesive, a plastic, a cosmetic, a surfactant, a detergent alcohol, a lubricant, a plasticizer, a flavor, and a fragrance.

## Description

This invention relates to a method of, and system and catalyst for esterification. The invention also provides for a method of making the catalyst capable of esterification, a catalyst composition, and systems using these catalysts, as well as esters made using the methods described herein, and downstream products derived from these esters.

Important esters used commercially are acetate esters, acrylic esters, natural fatty acid esters, sorbitol derived esters phthalates and benzoates and esters derived from maleic anhydride. However, most of the catalysts in the art have not been capable of esterification at high temperatures to produce esters with high selectivity. There exists a need for thermally stable catalysts capable of esterification at high temperatures and which produce an ester with high selectivity.

U.S. Patent No. 5,426,199 describes an esterification reaction which uses, as a catalyst, a styrene divinylbenzene resin which is surface functionalized with sulfonic acid groups. One problem with this technique is that the maximum operating temperature is only 130°C. If one proceeds above this temperature desulfonation of the catalyst occurs. British Patent No. 1,393,594 describes a styrene divinyl benzene resin which is core halogenated. Unfortunately, chlorinated polymers split off HCl in addition to sulfuric acid during use and lead to corrosion of stainless steel reactors and/or halide contamination of products.

There exists a significant need in the art for methods of esterification, systems, and catalysts that can be used for esterification that are thermally stable and provide improved performance characteristics, i.e. high selectivity for ester product. The present invention solves the problems in the art and provides high temperature, high selectivity methods of and systems for esterification, and catalysts that can be used for esterification that are thermally stable and provide improved performance characteristics. In addition to solving other problems in the art, the present invention also provides esters made using the methods and catalysts described herein as well as down stream products derived using these esters.

The invention provides a method of esterification that includes adding to reactants of a reaction a base treated, sulfonated, halogenated thermally stable catalyst. The catalyst includes a styrene DVB copolymer, a styrene DVB/EVB copolymer, or a combination thereof.

In another variant, the method includes a reaction used to prepare an acetate ester, an acrylic ester, a natural fatty acid ester, a sorbitol derived ester, a phthalate, and/or a benzoate and ester derived from maleic anhydride.

In yet another variant, the method is utilized in a system and manufacturing facility for esterification.

In yet another variant a downstream product made from an ester manufactured utilizing the method and system is also provided herein. Exemplary products include a solvent for a surface coating, an ink, a textile, an adhesive, a plastic, a cosmetic, a surfactant, a detergent alcohol, a lubricant, a plasticizer, a flavor, and a fragrance.

These and other variants of the invention are described in the remaining portions of the specification.

The catalytic activity of a chlorinated versus non-chlorinated styrene DVB (divinyl benzene) sulfonated polymer for the esterification reaction comprising ethanol and acetic acid was compared. Surprisingly, when using the same temperature, the chlorinated resin was more active than the unchlorinated resin. Moreover, a series of resins have been prepared that are chlorinated but provide minimal leaching of HCl under reaction conditions. The present invention concurrently provides resin catalysts having higher activity than catalysts in the art while simultaneously providing low leaching levels of chlorine into reactor systems. Use of these resins results in minimal reactor corrosion, and provides for substantial cost savings versus the use of conventional resins.

In one aspect, the present invention relates to a method of and system for esterification using a catalyst that includes a styrene DVB copolymer, and/or a DVB and EVB (ethyl vinyl benzene) copolymer that is sulfonated. Esters can be prepared by the following methods: a) the reaction of an alcohol with a carboxylic acid b) reaction between an olefin and a carboxylic acid and c) reaction between an anhydride and an alcohol to produce dialkyldiesters and d) via the transesterification of alcohols with esters. The method of this invention includes preparing an ester by methods a) through d) using a base treated, sulfonated or surface sulfonated, halogenated and acid regenerated thermally stable catalyst. In several variants, the esterification includes preparing acetate esters, acrylic esters, natural fatty acid esters, sorbitol derived esters, phthalates and benzoates, and esters derived from maleic anhydride.

In yet a further aspect, the invention provides an improved styrene DVB resin catalyst comprising 4-chlorostyrene aromatic groups and a polymer backbone. At least one of the aromatic groups has a chlorine in a styrenic para-position, and optional other chlorines thereon. The polymer backbone is substantially free of leachable chlorine. The catalyst includes a halogenated DVB moiety. The aromatic rings are optionally oleum sulfonated. The styrene DVB resin may also include optional sulfone bridges, and 4-halo (F,Br, and/or I) styrene.

The invention also provides a method of making a thermally stable catalyst that is used in esterification reactions. The method includes base treating a sulfonated and halogenated copolymer to obtain a base treated copolymer, and regenerating the base treated copolymer with an acid. In another variant, the method of making a thermally stable catalyst for esterification includes base treating a halogenated copolymer to obtain a base treated copolymer, and sulfonating the base treated copolymer.

Leachable chlorine is removed from the polymer backbone of the catalyst by the base treatment. Manufacturing methods using the catalyst described herein have the advantage of reduced levels of corrosion of equipment used for esterification versus conventional catalysts since there is significantly reduced leachable chlorine on the polymer backbone of the catalyst described herein. It is appreciated that significant cost savings are achieved and equipment longevity is increased through the use of the method, catalyst, and systems that use the method and catalyst described herein. Moreover, in one variant of the invention a polymer backbone is substantially free of leachable chlorine. There are no, substantially reduced, or very limited corrosion effects on equipment and/or prolonged life of the equipment as a result of use of the catalyst and system described herein.

The method of making a thermally stable catalyst for esterification optionally includes base treating a sulfonated and halogenated copolymer to obtain a base treated copolymer, and regenerating the base treated copolymer with an acid. The method of making a thermally stable catalyst for esterification optionally includes chlorinating a styrene DVB copolymer, base treating the halogenated copolymer, and sulfonating the halogenated and base treated catalyst. The styrene DVB copolymer is a polysulfonated copolymer in another variant of the invention.

In yet a further aspect, the invention provides an improved styrene DVB resin catalyst comprising aromatic groups having more than one SO₃H moiety and a polymer backbone. The improvement includes halogenated aromatic groups. The polymer backbone is substantially free of leachable chlorine. The catalyst is capable of high temperature esterification, while concurrently the leachable chlorine is at least an order of magnitude less than the leachable chlorine of a catalyst not base treated. In yet a further variant, the polymer backbone of the catalyst is free of leachable chlorine or other detrimental halogens.

It is a further benefit that the compressibility of a styrene DVB monosulfonated catalyst is decreased resulting in improved performance of the catalyst in manufacturing systems. The method includes increasing the number of chlorines on aromatic rings of the catalyst as compared to unhalogenated catalysts.

The first reaction scheme for making a catalyst of the present invention involves the following process: Styrene DVB copolymer --→ Sulfonate or surface sulfonate -→ chlorinate (or halogenate) -→ base treat --→ regenerate with acid.

The chlorination (or halogenation) step incorporates chlorine (or other halogen) in the aromatic ring as well as the aliphatic backbone of the polymer. It is the chlorine (or other halogen) on the backbone that can undesireably leach off slowly as HCl (or other corrosive compound) in an esterification process. Where this happens, accelerated equipment corrosion occurs resulting in undesirable down time and equipment replacement costs. Heating the polymer in a basic solution results in, for example, the leachable chlorine being removed. Moreover, this process can be carried out in the space of a several hours in contrast to the art process which takes 10 days to carry out, e.g. U.S. Patent No. 4,705,808. It is appreciated that the time needed to manufacture a catalyst of the invention is greatly reduced to the range of several hours, e.g. 1-10 hours, in one variant of the invention.

The final polymer has a higher acid site density than that created by prior methodologies. The current process does not have the drawback of extensive desulfonation in addition to chlorine removal. Consequently, a catalyst created by the process used herein has the advantage of lower desulfonation than conventional catalysts, in particular when operating at higher esterification temperatures. This results in higher catalytic activity since more active sites remain on the catalyst than after preparation using conventional processes. This also permits the catalyst and system to operate for longer periods of time and at higher temperatures before catalyst breakdown occurs. It is further appreciated that the number of catalyst change outs in the system will be reduced using the present invention, resulting in longer overall process times.

Scheme 1 was used to chlorinate Amberlyst™ 39 copolymer, a commercially available sulfonated styrene DVB(7%) co-polymer from Rohm and Haas Company. Other suitable resins could also be used herein. After refluxing with a 2N sodium hydroxide solution for 22 hours, followed by regeneration with hydrochloric acid, a new resin(**A**) was obtained. The hydrolytic stability of this resin was tested at 200°C for 24 hours. Table 1 shows that Resin A loses 9.5% of its sulfonic acid groups versus 33.4% lost by resin **F** which is a conventional chlorinated resin containing 12% DVB. Additionally, the percentage chlorine detected in solution is much lower for resin **A** than some of the other examples. We also subjected resin **F** to a base treatment to get resin **B** which has improved stability and lower chlorine leaching than resin **F**. Interestingly, resin **F** was also post treated using the process described in U.S. Patent No. 4,705,808 to get resin **C**. This patent desribes a process for the treatment of a halogenated strongly acidic cation exchange resin catalyst on a styrene-divinylbenzene copolymerizate base which comprises treating the catalyst with deionized water at a temperature ranging from about 100°C to about 150°C in the absence of oxygen and metal ions. It is clear from Table 1 that resin **B** has a higher acid capacity and is different from resin **C** in its properties and performance characteristics. Finally, scheme 1 was used to prepare resin **G** which is surface sulfonated and chlorinated, base treated and acid regenerated. The variant of the invention of scheme 1 involves a chlorination step followed by base treatment prior to the sulfonation. Additionally, the base treatment could be performed in any other solvent or mixtures of solvents than those described herein. The optimum conditions are resin specific, and can readily be determined empirically. In the examples described, a 50% aqueous NaOH solution for 4 hrs at 130°C was used.

Scheme 2 is also used to obtain a catalyst of the present invention suitable for improved performance in esterification reactions versus conventional resins: Styrene DVB copolymer → polysulfonate using oleum → chlorinate (or otherwise halogenate) → base treat → regenerate with acid.

Oleum sulfonation of a styrene DVB co-polymer results in a polysulfonated polymer ( one that has greater than one SO₃H group per ring). It is appreciated that the resin created by the process is both a polysulfonated and a chlorinated resin. Amberlyst™ 36 resin is a polysulfonated resin with 12% DVB. This resin was chlorinated and base treated as in Scheme 2 to get resin D. The thermal stability results of resin D (see Table 1) shows that it has high acid capacity, good stability and low chlorine leaching.

Scheme 3 is also used to obtain a catalyst of the present invention: 4-chlorostyrene DVB copolymer -→ sulfonate using oleum -→ chlorinate (or otherwise halogenate)-→ base treat→ regenerate with acid

A monomer and/or polymer is prepared using the process described in British Patent No. 1,393,594. A halogenated monomer such as chlorostyrene (Ortho-, meta-, para-, and/or combination thereof) with a crosslinker such as divinylbenzene is used as described in this patent, thereafter one sulfonates the polymer using chlorosulfonic acid. This polymer is still unstable at high temperatures because the DVB part is not deactivated by a halogen atom. In this variant of the invention, this polymer is chlorinated even further creating very desirable catalytic properties as well as solving the temperature stability problem resulting in a catalyst having high temperature stability.

In another variant of the invention, a copolymer of 4-chlorostyrene with 12% DVB was prepared and then monosulfonated using oleum as the sulfonating agent for relatively short reaction times. This is a substantial improvement on the procedure described in British Patent No. 1,393,594 resulting in a catalyst having substantially improved performance characteristics, e.g. the resulting resin has sulfone bridges which increase thermal stability. The resin was then further chlorinated and then base treated to remove leachable chlorine and after acid regeneration yielded resin **E**. The thermal stability of this resin and the low chlorine leaching coupled with the high acid capacity provides the resin with important catalytic properties and unexpected performance characteristics. Scheme 3 can therefore be used to prepare a variety of styrene DVB polymers that are fully halogenated and have SO₃H groups in a meta position. This method can be also used to prepare polysulfonated chlorinated resins as in Scheme 2 by varying the sulfonation conditions. In addition to this, the chlorination of the copolymer and base treatment can be performed prior to the sulfonation step.

It is further appreciated that the steps described in the various schemes mentioned above can be combined in various permutations such that the desired catalytic properties of the catalyst are obtained.

**TABLE 1**

| RESIN | %Cl %S | MmolSO3H/ g | Mmol SO3H/g after 200C, 24hours | % loss in acid sites | % chlorine in liquid after 200C, 24 hours |
|---|---|---|---|---|---|
| **A** | 26.78, 8.62 | 2.75 | 2.49 | 9.45 | 0.002 |
| **B** | 19.62, 10.24 | 3.31 | 2.50 | 24.60 | 0.19 |
| **F** | 22.00 | 3.14 | 2.10 | 33.40 | 1.34 |
| **C** | 19.30 | 3.06 | 2.12 | 30.40 | 0.08 |
| **D** | 10.04, 16.69 | 4.78 | 3.79 | 20.70 | 0.11 |
| **E** | 15.96, 13.95 | 4.16 | 3.52 | 15.34 | 0.12 |
| **G** | | 0.55 | 0.29 | 46.4 | 0.004 |

### COMPRESSIBILITY OF RESINS

Chlorination (or other halogenation) of a monosulfonated styrene DVB resin reduces its compressibility. The change in the compressibility depends on the percent DVB in resin and on the degree of chlorination or other halogenation. The results are shown below.

| RESIN | COMPRESSIBILITY cm/m |
|---|---|
| Styrene 12% DVB copolymer monosulfonated | 4.03 |
| Styrene 12% DVB copolymer chlorinated to 20% by weight | 3.03 |
| Styrene 7% DVB copolymer monosulfonated | 7.23 |
| Styrene 7% DVB copolymer monosulfonated and chlorinated to 27% by weight | 4.39 |

### CATALYTIC ACTIVITY OF RESINS

The catalytic activity of the halogenated resins for esterification reactions was studied using the reaction of excess ethanol with acetic acid at 60°C. The amount of dry resin added was adjusted so as to compare each resin on equal proton basis and the results are shown below.

**Table 2**

| **Resin** | **MmolH+/gram** | **%DVB** | **Dry wt of resin(g)** | **% conversion after 1 h** |
|---|---|---|---|---|
| Amberlyst™ 35 | 5.44 | 18.5 | 6.31 | 53.0 |
| Amberlyst™ 35 post chlorinated | 4.81 | 18.5 | 7.14 | 60.0 |
| Styrene 12% DVB sulfonated | 5.05 | 12 | 6.80 | 58.0 |
| Post chlorinated | 3.24 | 12 | 10.60 | 67.2 |

The results from Table 2 shows that chlorination increases the activity dramatically. We therefore conclude from Table 1 and 2 that A-E, G are excellent catalysts for esterification reactions having a combination of high thermal stability, activity, selectivity, and have lower corrosive tendencies. The temperature range in which the catalysts operate can be from 20-200°C. Resins A-E, and G are used as catalysts to prepare commercially important esters, by way of example, acetate esters, acrylic esters, natural fatty acid esters, sorbitol derived esters phthalates and benzoates and esters derived from maleic anhydride. Of course, other esters than the exemplary esters described above can also be prepared using the methods, catalysts and systems of the present invention.

### Example 1

Polymer synthesis: Styrene DVB co-polymers used to make catalysts of the present invention are commercially available in sulfonated forms. For example, Amberlyst ™ 39 (the Amberlyst ™ line is commercially available from Rohm and Haas Company) was chlorinated to make resin **A** using Scheme 1. Similarly, Amberlyst ™ 16 was used to prepare resin **F.** Treatment of resin **F** with water at 150 degrees C for ten days according to U.S. Patent No. 4,705,808 produced resin **C.** Amberlyst ™ 36 was chlorinated to make resin **D** according to Scheme 2. For resin E, the substrate 4-chlorostyrene co-polymer used to make a catalyst of the present invention was according to the procedure described in British Patent No. 1,393,594. The sulfonation was conducted using oleum at 110°C for 1 hour - 100g of polymer requires 1000g of oleum.

### Example 2

Chlorination of polymer: The following procedure is representative for all examples and chlorinations. However, it is appreciated that other chlorination procedures can be used to obtain the catalysts described herein. In a two liter glass reactor connected to a water circulation bath is placed 490 ml of wet Amberlyst ™ 36. To this is added 1180 g of water, and after stirring the water circulation bath is set to 35°C. The system is purged out for ten minutes with nitrogen and then chlorine is introduced into the reactor at 15psig (1.0549 kg/cm). The chlorine feed rate is a function of reaction rate and can be monitored by measuring weight loss. The percent HCl in the liquid is a measure of how much chlorine is on the resin since each Cl atom on the resin produces one HCl which dissolves in the water. This serves as a tool to gauge how much chlorine is on the resin. Aliquots of solution can be removed and titrated with base to measure percentage HCl in solution. After 15 hours, the percentage HCl was found to be 2 percent. The reaction was stopped and the resin is washed with several rinses of DI (dionized) water then sent for analysis: found Cl : 11.87%, S: 16.66%.

### Example 3

The following experiment illustrates that base treatment removes leachable chlorine: 75 grams of wet resin F was mixed with 220ml of 50% aqueous NaOH and heated for 4 hours at 130°C. The resin was filtered and the filtrate plus washes were weighed. A 10 g sample of the filtrate was treated with nitric acid to a pH of 2, then titrated with silver nitrate. Based on this result the amount of chlorine in 10gm of filtrate was normalized to the overall weight of filtrate plus washes. The amount of leachable chlorine in 75 g of wet resin was determined as 1.46g. The same experiment when repeated at room temperature for ten minutes gave only 0.47g of chlorine showing that the heat treatment with base removes additional chlorine from the polymer.

### Example 4

The following base treatment procedure was used to make resins **A, B, D, E.** Approximately 275 grams of chlorinated resin (washed with 1500 ml of deionized water) was placed in a 3 neck round-bottomed flask equipped with a mechanical stirrer, water condenser and a Thermowatch ™ device. To this was added 1192 ml of a 2N NaOH solution. The mixture was stirred and heated to reflux (103°C) for a total of 22 hrs. The solution was separated from the resin and combined with a 100 ml wash of the resin. The solution was acidified with HNO₃ and then titrated with silver nitrate for chloride determination. The resin was washed 3 times with 500 ml of deionized water then transferred to a column and washed with 3 liters of water (3 hrs) and 3 liters of 4% hydrochloric acid (3hrs) and then three liters of water(3hrs).

For resin **A** - the following was found before base treatment: 29.7% Cl, 8.2% S. After base treatment, the following results were obtained: 26.78% Cl, 8.62% S. The calculated amount of chloride recovered from the resin is 6.79g which is consistent with the reported analysis. For resin **D** - the following was found before base treatment: 11.87% Cl, 16.66% S. After base treatment, the following results were obtained: 10.04% Cl, 16.69% S. For resin **E** - the following was found before base treatment: 18.87% Cl, 13.97% S. After base treatment, the following results were obtained: 15.96% Cl, 13.95% S.

### Example 5

The following example gives one variant of a thermal stability test procedure used herein. In a 125 ml acid digestion bomb is added 40 ml of resin and 28 ml of deionized water. The bomb is sealed and placed in a vacuum oven which is then heated to 200 degrees C. After 24 hours, the heat is turned off and the oven is cooled for 4 hours. The bomb is removed and the lid is removed in a hood. The liquid is separated from the resin and the pH is measured and the chlorine content determined by titration. The resin is washed thoroughly and its acid capacity is measured as described in example 6. The same experiment was also repeated in three smaller digestion bombs, each containing 14 ml of resin and 10 ml of water. After the thermal stability experiment the three samples were combined for analysis. Results are shown in Table 1.

### Example 6

The following procedure is a general method used to determine acid capacity of all resins. The acid capacity of resin can be determined as follows: The wet resin is placed in a beaker and dried overnight at 110°C. The beaker is placed in a dessicator allowed to cool and weighed. The first weight observed on the balance is recorded (allowing the resin to sit exposed to the air results in reabsorption of moisture). The weight of dry solid is recorded (typically we used between 5.5 and 6.0g). After transferring to a column the resin, approximately 300 ml of deionized water is passed through the resin for half hour. Separately, a solution of sodium nitrate is prepared by dissolving 45 g in a liter of water. This flows through the column in 2 hours and the eluate is collected in a 1000 ml volumetric flask up to the mark. A 100 ml portion of this sample is titrated with 0.1008 N solution of sodium hydroxide. The acid capacity is calculated as [V1*0.1008*10/W1] mmol H+ per gram of dry resin where V1 is the volume of base required for neutralization and W1 is the dry weight of resin.

### Example 7

In a round bottomed flask fitted with a water cooled condenser and mechanical stirrer was added 200ml of ethanol and 100 ml of acetic acid. The contents were heated to 70°C at which time the dry catalyst was added to the flask with stirring. After 1 hour an aliquot was removed and analyzed by GC and also titrated with base to determine acetic acid conversion. The conversions are reported in Table 2.

### Example 8

This is an example of a high temperature esterification and uses a similar example as in U.S. Patent No. 6,274,749. Palmitic acid 522g and 265g of octanol is heated with 100 g of catalyst chosen from A-E, G to 190°C in a stirred vessel. The progress of reaction is followed by distilling off the water formed as product.

### Example 9

A process similar to that used in U.S. Patent No. 4,332,738 is used in this example. One mole of alcohol is reacted with one mole of neo acid(pentanoic) or decanoic acid in the presence of catalysts A-E, and G. The temperature is selected between 50-170°C and the reaction is followed by IR until complete.

### Example 10

An acid anhydride such as maleic or phthalic is reacted with an excess alcohol at temperatures between 100-250°C containing up to 10 wt % of catalyst chosen from A-E, G. The reaction is conducted so as to reflux alcohol and byproduct water which is removed. Using this method important compounds such as dibutyl and dioctyl maleate and dioctyl phthalate are prepared.

Down stream products that are made from the esters manufactured using the present invention include, for example: solvents for surface coating, inks, textiles, adhesives, plastics, cosmetics, surfactants, lubricants, plasticizers, flavors, and fragrances. It is appreciated that the foregoing list is not exhaustive and that other downstream products that are made from or use esters as components thereof are also contemplated as downstream products. By way of further example, downstream products include products which are made from or include acetate esters, acrylic esters, natural fatty acid esters, sorbitol derived esters, phthalates and benzoate,s and esters derived from maleic anhydride.

While only a few, preferred embodiments of the invention have been described hereinabove, those of ordinary skill in the art will recognize that the embodiment may be modified and altered without departing from the central spirit and scope of the invention. Thus, the preferred embodiment described hereinabove is to be considered in all respects as illustrative and not restrictive, the scope of the invention being indicated by the appended claims, rather than by the foregoing description, and all changes which come within the meaning and range of equivalency of the claims are intended to be embraced herein.

## Claims

1. A method of esterification, comprising:
using a base treated, sulfonated, halogenated thermally stable catalyst, said catalyst comprising a styrene DVB copolymer, a styrene DVB/EVB copolymer, or a combination thereof, in an esterification reaction.

2. The method as claimed in claim 1 wherein said catalyst is surface sulfonated.

3. The method of claim 1 in which said catalyst is acid regenerated.

4. The method as claimed in claim 1 wherein the reaction takes place at temperatures in the range of 60-200°C.

5. The method as defined in claim 1 wherein the reaction is a reaction used to prepare an acetate ester, an acrylic ester, a natural fatty acid ester, a sorbitol derived ester, a phthalate, and/or a benzoate and ester derived from maleic anhydride.

6. A system for esterification utilizing the method of claim 1.

7. An ester manufactured utilizing the method of claim 1.

8. The ester of claim 7 in which said ester is selected from the group consisting of an acetate ester, an acrylic ester, a natural fatty acid ester, a sorbitol derived ester phthalate, a sorbitol derived ester benzoate, and an ester derived from maleic anhydride.

9. A downstream product made from an ester manufactured utilizing the method of claim 1.

10. The downstream product of claim 9 in which said product is selected from the group consisting of a solvent for a surface coating, an ink, a textile, an adhesive, a plastic, a cosmetic, a surfactant, a detergent alcohol, a lubricant, a plasticizer, a flavor, and a fragrance.
